(19) **Europäisches Patentamt**

**European Patent Office**

**Office européen des brevets**

(11) Numéro de publication: **0 304 347 B1**

(12) # FASCICULE DE BREVET EUROPEEN

(45) Date de publication de fascicule du brevet: **08.12.93**

(51) Int. Cl.5: **C08B 37/00**, A61K 35/78, A23K 1/16

(21) Numéro de dépôt: **88401774.0**

(22) Date de dépôt: **07.07.88**

(54) **Polysaccharides extraits, notamment, de végétaux utiles comme médicaments et additifs alimentaires.**

(30) Priorité: **10.07.87 FR 8709857**

(43) Date de publication de la demande:
**22.02.89 Bulletin 89/08**

(45) Mention de la délivrance du brevet:
**08.12.93 Bulletin 93/49**

(84) Etats contractants désignés:
**AT BE CH DE ES FR GB GR IT LI LU NL SE**

(56) Documents cités:
**FR-A- 1 520 375**
**FR-A- 2 513 518**

**S.K. JAIN: "Medicinal plants", chapitre 34, "Euphorbia", pags 67-69, National Book Trust India**

**CARBOHYDRATE RESEARCH, vol. 89, 1981, pages 137-143, Elsevier Scientific Publishing Co., Amsterdam, NL; A.J. BUCHALA et al.: "An arabinogalactan from the fibres of cotton (Gossypium arboreum L.)"**

(73) Titulaire: **GUYOMARC'H NUTRITION ANIMALE Talhouet**
**F-56250 Saint-Nolff(FR)**

(72) Inventeur: **Nguyen, Tan Hung**
**Le Porlair**
**F-56890 Saint Ave(FR)**

(74) Mandataire: **Martin, Jean-Jacques et al**
**Cabinet REGIMBEAU**
**26, Avenue Kléber**
**F-75116 Paris (FR)**

**Description**

La présente invention a pour objet des polysaccharides extraits de végétaux, ainsi que des procédés pour leur extraction et leur application à titre de médicaments et d'additifs alimentaires.

Ces nouvelles substances polysaccharidiques ont la propriété d'augmenter la sécrétion de la prolactine, principale hormone de la lactation, et d'augmenter la sécrétion de la béta-caséine, principale protéine du lait, et, par voie de conséquence, augmentent la sécrétion de lait chez les animaux et les êtres humains, et sont donc susceptibles de recevoir des applications industrielles, notamment dans l'élevage des animaux et dans l'allaitement maternel de l'espèce humaine.

Plus particulièrement, la présente invention concerne des polysaccharides contenant des unités correspondant au rhamnose, arabinose, xylose, mannose, galactose et glucose assurant chez les animaux et les humains une augmentation du taux de prolactine plasmatique.

Ces polysaccharides sont, en outre, caractérisés en ce que leur masse moléculaire est comprise entre $10^4$ et $10^6$ daltons, en général entre $2.10^4$ et $10^5$ daltons.

Ces polysaccharides contiennent, en outre, de l'azote et du soufre, en général de 1 à 8 % d'azote et de 0,8 à 2 % de soufre.

Ces produits sont d'origine végétale, mais il est possible d'envisager leur synthèse ou leur hémi-synthèse à partir de produits d'origine diverse ou même une synthèse totale à partir de produits chimiques connus.

L'invention concerne également des procédés de préparation de ces polysaccharides à partir de plantes contenant ces polysaccharides par extraction à l'eau chaude, les polysaccharides étant récupérés à partir de l'extrait aqueux obtenu, celui-ci pouvant éventuellement être purifié.

La purification peut être effectuée par tous les procédés connus, mais en particulier il est possible de prévoir une précipitation de l'extrait aqueux à l'aide de solvants organiques miscibles à l'eau et ne dissolvant pas les polysaccharides, en particulier les solvants hydroxylés comme l'éthanol, le méthanol.

Il est bien entendu possible de prévoir d'autres techniques de purification telles que des fractionnements par poids moléculaires.

Les produits selon la présente invention sont extraits de plantes du genre Gossypium sp. de la famille des Malvacées et de l'espèce Euphorbia Hirta Linné de la famille des Euphorbiacées.

Le procédé de l'invention consiste essentiellement à traiter des organes végétaux de Gossypium sp., en particulier les graines réduites en poudre ou les tourteaux correspondants. Pour l'Euphorbia Hirta, c'est la plante entière qui est réduite en poudre. La poudre dégraissée ou non au préalable est extraite par de l'eau à chaud, ce qui fournit une solution aqueuse. La solution aqueuse est concentrée et traitée de différentes façons.

La solution aqueuse concentrée est soit lyophilisée soit transformée en une poudre sèche par des méthodes physiques d'évaporation et de séchage.

La poudre ainsi obtenue est traitée ensuite soit par de l'éthanol, soit par du méthanol, soit par d'autres solvants organiques ne dissolvant pas les polysaccharides.

La solution aqueuse d'extraits totaux peut être aussi traitée par de l'éthanol ou d'autres solvants pour précipiter les polysaccharides totaux.

A la fin des opérations précitées, on obtient l'ensemble des fractions polysaccharidiques galactogènes. Si nécessaire, une purification plus poussée peut être obtenue par des méthodes physiques, chimiques ou physico-chimiques de fractionnement connues : ultrafiltration, filtration sur gel, précipitation différentielle, chromatographie par affinité, par exemple.

La présente invention concerne plus particulièrement les fractions de nature polysaccharidique, hautement actives, extraites du genre Gossypium sp. nommées Gossypine A et Gossypine B.

Les Gossypines A et B sont caractérisées de la façon suivante :

Les Gossypines A et B sont des polysaccharides, de masse moléculaire comprise entre $2.10^4$ - $10^5$ daltons.

La Gossypine A correspond à la fraction de masse moléculaire comprise entre $2.10^4$ et $5.10^4$ daltons et contient 3,40 % ± 0,3 d'N et 1,32 % ± 0,3 de S.

Par hydrolyse acide, la Gossypine A fournit des monosaccharides qui sont transformés en alditols par réduction au borohydrure de sodium. Ces monosaccharides réduits sont acétylés et analysés par la technique de chromatographie en phase gazeuse (CPG) couplée à la spectrométrie de masse (SM). La Gossypine A fournit les résultats (en analyses par CPG - SM) en pourcentage de monosaccharides :

2

| Rha(*) | Ara | Xyl | Man | Gal | Glu | dérivé acétylé |
|--------|-----|-----|-----|-----|-----|----------------|
| 1,1 % | 5,7 % | 1,5 % | 7,6 % | 40,6 % | 41,8 % | 1,7 % |

(*) On admettra par convention que les symboles Rha, Ara, Xyl, Man, Gal, Glu représentent les dérivés réduits et acétylés des oses correspondants: Rhamnose, Arabinose, Xylose, Mannose, Galactose, Glucose.

La chromatographie en phase gazeuse est réalisée d'après les conditions suivantes :

Colonne capillaire en verre de longueur 25 m, de diamètre 0,32 m/m, épaisseur de film : 0,2 $\mu$m ; phase : Silar 10 C, température de colonne : 220°C, température de l'injecteur : 220°C, température de l'interface : 250°C, pression de l'hélium : 0,7 Bar.

La spectrométrie de masse est réalisée à 70 eV - 0,25 $\mu$A, photomultiplicateur 1 300 eV.

La Gossypine B correspond à la fraction de masse moléculaire comprise entre $5.10^4$ et $10^5$ daltons et contient 6,7 % ± 0,3 d'N et 1,32 % ± 0,3 de S.

Par hydrolyse acide, la Gossypine B fournit des monosaccharides qui sont analysés sous forme de dérivés alditols, dans les mêmes conditions que dans le cas de la Gossypine A.

La Gossypine B, en analyse par CPG - SM correspond au pourcentage suivant de monosaccharides :

| Rha | Ara | Xyl | Man | Gal | Glu | dérivé acétylé |
|-----|-----|-----|-----|-----|-----|----------------|
| 3,1 % | 16,3 % | 6,1 % | 2,5 % | 33,9 % | 33,3 % | 4,8 % |

La présente invention concerne également les fractions actives, galactogènes, extraites du genre Euphorbia Hirta Linné, nommées EUPHORBINE A et EUPHORBINE B.

Les Euphorbines A et B sont caractérisées de la façon suivante :

Les Euphorbines A et B sont des polysaccharides de masse moléculaire comprise entre $3.10^4$ et $10^5$ daltons.

L'Euphorbine A correspond à la fraction de masse moléculaire comprise entre $3.10^4$ et $5.10^4$ daltons et contient 3,10 % ± 0,3 de N et 1,09 % ± 0,3 de S.

Par hydrolyse acide, l'Euphorbine A conduit à des monosaccharides qui sont transformés sous la forme réduite des oses. Ces monosaccharides réduits sont acétylés et analysés par la technique de chromatographie en phase gazeuse, couplée à la spectrométrie de masse (CPG-SM).

Par CPG - SM dans les mêmes conditions que dans le cas des Gossypines A et B, l'Euphorbine A fournit les résultats suivants :

| Rha | Ara | Xyl | Man | Gal | Glu | dérivé acétylé |
|-----|-----|-----|-----|-----|-----|----------------|
| 8,4 % | 19,5 % | 4,2 % | 3,8 % | 19,3 % | 42,6 % | 2,2 % |

L'Euphorbine B est une fraction de polysaccharide galactogène, de masse moléculaire comprise entre $5.10^4$ et $10^5$ daltons et contient 2,5 % ± 0,3 de N et 1,1 ± 0,3 de S.

Par hydrolyse acide, l'Euphorbine B conduit à des monosaccharides qui sont transformés sous la forme réduite des oses ou alditols. Les alditols obtenus sont acétylés et analysés par la technique (CPG-SM).

L'Euphorbine B correspond aux résultats suivants :

| Rha | Ara | Xyl | Man | Gal | Glu | dérivé acétylé |
|-----|-----|-----|-----|-----|-----|----------------|
| 12,7 % | 25,1 % | 9,5 % | 9,2 % | 15,8 % | 19,2 % | 8,4 % |

Les produits selon la présente invention sont plus particulièrement intéressants pour augmenter le taux de prolactine plasmatique chez les animaux ou chez les êtres humains. Chez les mammifères ils augmentent le taux de béta-caséine de la glande mammaire.

EP 0 304 347 B1

Ces propriétés galactogènes peuvent être mises à profit dans l'élevage des animaux et dans l'allaitement maternel de l'espèce humaine, tant sous forme de médicament que sous forme d'additif alimentaire.

Les exemples ci-après permettront de mettre en évidence d'autres caractéristiques et avantages de la présente invention.

## EXEMPLE 1

A 1 000 g de plantes entières pulvérisées de Euphorbia Hirta, on ajoute 6 litres d'eau. Le mélange est porté à 90-95°C pendant 20 minutes. On filtre, le résidu solide est traité de la même façon, deux fois de suite. Les filtrats aqueux sont réunis et concentrés à 2 litres. Cet extrait aqueux est lyophilisé. Le lyophilisat obtenu est traité par de l'éthanol à reflux pendant 5 minutes (3 fois 500 ml). La partie insoluble pèse 98,1 g et constitue l'extrait total polysaccharidique.

## EXEMPLE 2

A 1 000 g de plantes entières pulvérisées de Euphorbia Hirta, on ajoute 6 litres d'eau, le mélange est porté à 90-95°C pendant 20 minutes. On filtre, le résidu solide est traité de la même façon, deux fois de suite. Les filtrats aqueux sont réunis et concentrés à 0,3 litre. On y ajoute 2 litres d'éthanol et on sépare le précipité. Le précipité séché pèse 101,5 g et constitue l'extrait total polysaccharidique.

## EXEMPLE 3

100 g d'extrait total polysaccharidique de Euphorbia Hirta sont dissous dans 2 litres d'eau. On filtre, le filtrat est filtré ensuite sur membrane à perméabilité sélective, à seuil d'arrêt nominal en poids moléculaire de $3.10^4$ daltons. La phase aqueuse contenant des produits de masse moléculaire inférieure à $3.10^4$ daltons est lyophilisée et fournit 21,4 g. La phase aqueuse contenant des molécules de masse moléculaire supérieure à $3.10^4$ daltons est ultrafiltrée sur une membrane à seuil d'arrêt de $10^5$ daltons. La phase aqueuse contenant des molécules de masses moléculaire comprise entre $3.10^4$ daltons et $10^5$ daltons est ultrafiltrée ensuite sur une membrane à seuil d'arrêt de $5.10^4$ daltons. Après lyophilisation, ou nébulisation, de ces fractions on obtient 8,3 g d'Euphorbine A et 2,2 g d'Euphorbine B.

## EXEMPLE 4

A 10 kg de poudre de graine de Gossypium sp. on ajoute 100 litres d'eau. Le mélange est porté à 90-95°C pendant 20 minutes. On filtre. Le résidu solide est traité deux fois de suite avec 70 litres d'eau chaque fois, à 90-95°C pendant 20 minutes. Les filtrats réunis conduisent à 2,14 kg de nébulisats de polysaccharides bruts.

## EXEMPLE 5

A 100 g de nébulisats de polysaccharides bruts de Gossypium sp. on ajoute 500 ml d'éthanol et le mélange est porté à reflux pendant 5 minutes. Le résidu est repris deux fois successivement avec de l'éthanol (2 x 250 ml). Le résidu insoluble dans l'éthanol (83,3 g) constitue l'extrait total polysaccharidique.

## EXEMPLE 6

A 10 kg de tourteaux de graines de Gossypium sp. on ajoute 100 litres d'eau. Le mélange est porté à 90-95°C pendant 20 minutes. On filtre, le résidu est traité de nouveau deux fois avec 70 litres d'eau chaque fois, à 90-95°C pendant 20 minutes. Les filtrats réunis fournissent 1,91 kg de nébulisats de polysaccharides bruts.

## EXEMPLE 7

A 100 g de nébulisat de polysaccharides bruts de Gossypium sp. de l'exemple 6, on ajoute 500 ml d'éthanol et le mélange est porté à reflux pendant 5 minutes. Le résidu solide est repris deux fois avec de l'éthanol (2 x 250 ml). Le résidu insoluble dans l'éthanol constitue l'extrait total polysaccharidique (90,1 g).

4

EXEMPLE 8

20 g de l'extrait total polysaccharidique de Gossypium sp. de l'exemple 7 sont dissous dans 5 litres d'eau. La solution aqueuse est ultrafiltrée successivement sur une membrane à seuil d'arrêt de $10^5$ daltons. La fraction de masse moléculaire inférieure à $10^5$ daltons est ultrafiltrée sur une membrane à seuil d'arrêt de $5.10^4$ daltons.

La fraction de masse moléculaire inférieure à $5.10^4$ daltons est ultrafiltrée sur une membrane à seuil d'arrêt de $2.10^4$ daltons. Ces différentes fractions aqueuses sont lyophilisées et on obtient ainsi 295 mg de Gossypine A de masse moléculaire comprise entre $2.10^4$ et $5.10^4$ daltons et 208 mg de Gossypine B de masse moléculaire comprise entre $5.10^4$ et $10^5$ daltons.

EXEMPLE 9

ETUDES PHARMACOLOGIQUES

Activité galactogène

Principe

L'administration à des animaux d'un produit galactogène se traduit part :
1) une augmentation du taux de béta-caséine de la glande mammaire ;
2) une augmentation du taux de prolactine plasmatique.

Dosage radio-immunologique de la béta-caséine

Des rates âgées de 3 moins sont réparties par lot de 4 rates. Le produit à étudier est, soit en suspension, soit en solution dans 1 ml d'eau, administré par gavage deux fois par jour pendant 4 jours. Au 5ème jour, les glandes mammaires des animaux sacrifiés sont prélevées pour le dosage radio-immunologique de la béta-caséine (selon la méthode de F.C. GREENWOOD, W.M. HUNTER et J.G. GLOVER, Bioch. J. (1963) 89, 114). De l'eau est administrée aux rates témoins. On évalue le rapport de la quantité de béta-caséine produite par les glandes mammaires des rates traitées et de celle produite par les glandes mammaires des rates témoins.

Les résultats obtenus sont les suivants :

| Dose/jour | Produits | Béta-caséine rates traitées/ Béta-caséine rates témoins |
|---|---|---|
| | Eau (témoin) | 1 |
| 400 mg | produit de l'exemple 4 | 3 |
| 400 mg | produit de l'exemple 5 | 4 |
| 400 mg | produit de l'exemple 6 | 3 |
| 400 mg | produit de l'exemple 7 | 4 |
| 200 mg | produit de l'exemple 1 | 2 |
| 10 mg | Gossypine A | 4 |
| 10 mg | Gossypine B | 5 |
| 10 mg | Euphorbine A | 2 |
| 10 mg | Euphorbine B | 4 |

Dosage radio-immunologique de la prolactine

On dose le taux de prolactine plasmatique des brebis ayant reçu, en injection intra-veineuse, des produits à étudier, utilisant le sérum physiologique comme excipient. Des prélèvements sanguins sont effectués toutes les 10 minutes et ceci pendant une heure. Le plasma obtenu par centrifugation sert au dosage radio-immunologique de la prolactine qui est exprimée en ng par ml de sérum. Les brebis sont leur propre témoin. On dose le taux de prolactine plasmatique avant et après l'injection des produits à étudier. Les résultats, exprimés par le rapport entre le taux maximum de prolactine obtenue après l'injection et le taux de base obtenu avant l'invention, son les suivants :

| Dose/jour | Produits | Prolactine |
|---|---|---|
| | sérum physiologique (témoin) | 1 |
| 500 mg | produit de l'exemple 4 | 4 |
| 300 mg | produit de l'exemple 1 | 4 |
| 100 mg | Gossypine A | 2 |
| 100 mg | Gossypine B | 5 |
| 200 mg | Euphorbine A | 4 |
| 50 mg | Euphorbine B | 5 |

**Revendications**

**1.** Polysaccharide d'origine végétale comprenant les unités correspondant au Rhamnose, Arabinose, Xylose, Mannose, Galactose, extrait de Gossypium sp et/ou de Euphorbia hirta, caractérisé en ce qu'il est choisi parmi la Gossypine A, la Gossipine B, l'Euphorbine A et l'Euphorbine B, répondant aux caractéristiques suivantes :
- **Gossypine A** : fraction de masse moléculaire comprise entre $2.10^4$ et $5.10^4$ daltons et contenant $\overline{3,40\%}$ +/- 0,3 de N et 1,32% +/- 0,3 de S,
- **Gossypine B** : fraction de masse moléculaire comprise entre $5.10^4$ et $10^5$ daltons et contenant $\overline{6,70\%}$ +/- 0,3 de N et 1,32% +/- 0,3 de S,
- **Euphorbine A** : fraction de masse moléculaire comprise entre $3.10^4$ et $5.10^4$ daltons et contenant $\overline{3,10\%}$ +/- 0,3 de N et 1,09% +/- 0,3 de S,
- **Euphorbine B** : fraction de masse moléculaire comprise entre $5.10^4$ et $10^5$ daltons et contenant $\overline{2,5\%}$ +/- de N et 1,1% +/- 0,3 de S.

**2.** Polysaccharide selon la revendication 1, caractérisé en ce que la Gossypine A est constituée du pourcentage suivant de monosaccharides :

| Rha | Ara | Xyl | Man | Gal | Glu | dérivé acétylé |
|---|---|---|---|---|---|---|
| 1,1% | 5,7% | 1,5% | 7,6% | 40,6% | 41,8% | 1,7% |

**3.** Polysaccharide selon la revendication 1, caractérisé en ce que la Gossypine B est constituée du pourcentage suivant de monosaccharide :

| Rha | Ara | Xyl | Man | Gal | Glu | dérivé acétylé |
|---|---|---|---|---|---|---|
| 3,1% | 16,3% | 6,1% | 2,5% | 33,9% | 33,3% | 4,8% |

**4.** Polysaccharide selon la revendication 1, caractérisé en ce que l'Euphorbine A est constituée du pourcentage suivant de monosaccharides :

EP 0 304 347 B1

| Rha | Ara | Xyl | Man | Gal | Glu | dérivé acétylé |
|---|---|---|---|---|---|---|
| 8,4% | 19,5% | 4,2% | 3,8% | 19,3% | 42,6% | 2,2% |

5.  Polysaccharide selon la revendication 1, caractérisé en ce que l'Euphorbine B est constituée du pourcentage suivant de monosaccharides :

| Rha | Ara | Xyl | Man | Gal | Glu | dérivé acétylé |
|---|---|---|---|---|---|---|
| 12,7% | 25,1% | 9,5% | 9,2% | 15,8% | 19,2% | 8,4% |

6.  Polysaccharide selon l'une des revendications 1 à 5, caractérisé en ce qu'il augmente chez les mammifères le taux de bêta-caséine de la glande mammaire.

7.  Procédé de préparation de polysaccharides selon l'une des revendications 1 à 6 caractérisé en ce que l'on effectue une extraction d'une plante contenant lesdits polysaccharides par de l'eau chauffée a 90-95°C pendant environ 20 minutes et éventuellement une précipitation de l'extrait aqueux contenant les polysaccharides, par un solvant organique miscible à l'eau et ne dissolvant pas les polysaccharides, et en ce que l'on sépare les polysaccharides ayant le poids moléculaire décrit dans la revendication 1.

8.  Médicament assurant, chez les animaux et les humains une augmentation du taux de prolactine plasmatique et contenant à titre de principe actif au moins un polysaccharide selon l'une des revendications 1 à 6.

9.  Additif alimentaire assurant, chez les animaux et les humains une augmentation du taux de prolactine plasmatique et contenant un polysaccharide selon l'une des revendications 1 à 6.

**Claims**

1.  Polysaccharide from plants extracted from Gossypium sp and/or Euphorbia hirta containing units corresponding to rhamnose, arabinose, xylose, mannose, galactose, characterized in that it is chosen from among :
    - . gossypine A
    - . gossypine B
    - . euphorbine A
    - . euphorbine B, responding to the following characteristics :
        Gossypine A : molecular mass fraction lying between $2.10^4$ and $5.10^4$ daltons and containing 3.40% ± 0.3 of N and 1.32% ± 0.3 of S,
        Gossypine B : molecular mass fraction lying between $5.10^4$ and $10^5$ daltons and containing 6.7% ± 0.3 of N and 1.32% ± 0.3 of S,
        Euphorbine A : molecular mass fraction lying between $3.10^4$ and $5.10^4$ daltons and containing 3.10% ± 0.3 of N and 1.09% ± 0.3 of S,
        Euphorbine B : molecular mass fraction lying between $5.10^4$ and $10^5$ daltons and containing 2.5% ± 0.3 of N and 1.1 ± 0.3 of S.

2.  Polysaccharide according to claim 1, characterized in that Gossypine A is constituted with the following percentages of monosaccharide :

| Rha | Ara | Xyl | Man | Gal | Glu | acetylated derivative |
|---|---|---|---|---|---|---|
| 1.1 % | 5.7 % | 1.5 % | 7.6 % | 40.6 % | 41.8 % | 1.7 % |

3.  Polysaccharide according to claim 1, characterized in that Gossypine B is constituted with the following percentages of monosaccharide :

7

| Rha | Ara | Xyl | Man | Gal | Glu | acetylated derivative |
|---|---|---|---|---|---|---|
| 3.1 % | 16.3 % | 6.1 % | 2.5 % | 33.9 % | 33.3 % | 4.8 % |

4. Polysaccharide according to claim 1, characterized in that Euphorbine A is constituted with the following percentages of monosaccharides :

| Rha | Ara | Xyl | Man | Gal | Glu | acetylated derivative |
|---|---|---|---|---|---|---|
| 8.4 % | 19.5 % | 4.2 % | 3.8 % | 19.3 % | 42.6 % | 2.2 % |

5. Polysaccharide according to claim 1, characterized in that Euphorbine B is constituted with the following percentages of monosaccharides :

| Rha | Ara | Xyl | Man | Gal | Glu | acetylated derivative |
|---|---|---|---|---|---|---|
| 12.7 % | 25.1 % | 9.5 % | 9.2 % | 15.8 % | 19.2 % | 8.4 % |

6. Polysaccharide according to one of claims 1 to 5, characterized in that it increases the level of beta-casein in the mammary gland of mammals.

7. Procedure for the preparation of polysaccharides according to one of the Claims 1 to 6, characterized in that an extraction with hot water at 90-95°C for 20 minutes is made of a plant containing the said polysaccharides and in that the aqueous extract containing the polysaccharides is eventually precipitated by an organic solvent miscible with water and which does not dissolve the polysaccharides, and in that the polysaccharides having the molecular weight described in claim 1 are separated.

8. Medicine providing animals and humans with an increased level of plasma prolactin, containing as active principle at least an polysaccharide according to one of claims 1 to 6.

9. Food additive providing animals and humans with an increased level of plasma prolactin containing a polysaccharide according to one of claims 1 to 6.

**Patentansprüche**

1. Polysaccharid pflanzlicher Herkunft, umfassend Einheiten, die Rhamnose, Arabinose, Xylose, Mannose oder Galactose entsprechen, extrahiert aus Gossypium sp und/oder Euphorbia hirta, dadurch gekennzeichnet, daß es ausgewählt wird unter Gossypin A, Gossypin B, Euphorbin A und Euphorbin B, die den folgenden Charakteristiken entsprechen:
   - Gossypin A: Fraktion mit der Molekularmasse zwischen $2 \cdot 10^4$ und $5 \cdot 10^4$ Dalton und enthaltend $\overline{3{,}40\ \%\ \pm\ 0{,}3}$ N und 1,32 % ± 0,3 S,
   - Gossypin B: Fraktion mit der Molekularmasse zwischen $5 \cdot 10^4$ und $10^5$ Dalton und enthaltend $\overline{6{,}70\ \%\ \pm\ 0{,}3}$ N und 1,32 % ± 0,3 S,
   - Euphorbin A: Fraktion mit der Molekularmasse zwischen $3 \cdot 10^4$ und $5 \cdot 10^4$ Dalton und enthaltend $\overline{3{,}10\ \%\ \pm\ 0{,}3}$ N und 1,09 % ± 0,3 S,
   - Euphorbin B: Fraktion mit der Molekularmasse zwischen $5 \cdot 10^4$ und $10^5$ Dalton und enthaltend 2,5 $\overline{\%\ \pm\ N\ und\ 1{,}1}$ % ± 0,3 S,

2. Polysaccharid nach Anspruch 1, dadurch gekennzeichnet, daß das Gossypin A prozentual aus den folgenden Monosacchariden besteht:

| Rha | Ara | Xyl | Man | Gal | Glu | Acetyl-Derivat |
|-----|-----|-----|-----|-----|-----|----------------|
| 1,1% | 5,7% | 1,5% | 7,6% | 40,6% | 41,8% | 1,7% |

**3.** Polysaccharid nach Anspruch 1, dadurch gekennzeichnet, daß das Gossypin B prozentual aus den folgenden Monosacchariden besteht:

| Rha | Ara | Xyl | Man | Gal | Glu | Acetyl-Derivat |
|-----|-----|-----|-----|-----|-----|----------------|
| 3,1% | 16,3% | 6,1% | 2,5% | 33,9% | 33,3% | 4,8% |

**4.** Polysaccharid nach Anspruch 1, dadurch gekennzeichnet, daß das Euphorbin A prozentual aus den folgenden Monosacchariden besteht:

| Rha | Ara | Xyl | Man | Gal | Glu | Acetyl-Derivat |
|-----|-----|-----|-----|-----|-----|----------------|
| 8,4% | 19,5% | 4,2% | 3,8% | 19,3% | 42,6% | 2,2% |

**5.** Polysaccharid nach Anspruch 1, dadurch gekennzeichnet, daß das Euphorbin B prozentual aus den folgenden Monosacchariden besteht:

| Rha | Ara | Xyl | Man | Gal | Glu | Acetyl-Derivat |
|-----|-----|-----|-----|-----|-----|----------------|
| 12,7% | 25,1% | 9,5% | 9,2% | 15,8% | 19,2% | 8,4% |

**6.** Polysaccharid nach einem der Ansprüche 1 bis 5, dadurch gekennzeichnet, daß es bei Säugetieren den Gehalt an Beta-Casein in den Milchdrüsen erhöht.

**7.** Verfahren zur Herstellung von Polysacchariden nach einem der Ansprüche 1 bis 6, dadurch gekennzeichnet, daß man die Extraktion einer Pflanze, die die genannten Polysaccharide enthält, mit auf eine Temperatur von 90 °C bis 95 °C erwärmtem Wasser während etwa 20 Minuten durchführt, und man gegebenenfalls eine Fällung der die Polysaccharide enthaltenden wäßrigen Extrakte mit einem organischen, mit Wasser mischbaren Lösungsmittel, das die Polysaccharide nicht löst, vornimmt, und dadurch, daß man die Polysaccharide mit dem in Anspruch 1 genannten Molekulargewicht abtrennt.

**8.** Medikament, das bei Tieren und Menschen eine Erhöhung des plasmatischen Prolactin-Gehaltes gewährleistet und als Wirkstoff mindestens ein Polysaccharid nach einem der Ansprüche 1 bis 6 enthält.

**9.** Nahrungs-Zusatzstoff, der bei Tieren und Menschen eine Erhöhung des plasmatischen Prolactin-Gehaltes gewährleistet und ein Polysaccharid nach einem der Ansprüche 1 bis 6 enthält.